# EUROPÄISCHE PATENTSCHRIFT

(11) **EP 0 715 862 B1**
(45) Veröffentlichungstag und Bekanntmachung des Hinweises auf die Patenterteilung: **14.06.2000**
(21) Anmeldenummer: 95114866.7
(22) Anmeldetag: 21.09.1995
(51) Int. Cl.: A61M 16/00

(54) **Atemgasbehälter**
Breathing gas reservoir
Réservoir pour gaz respirable

(30) Priorität: 08.11.1994 DE 4439474
(43) Veröffentlichungstag der Anmeldung: 12.06.1996
(73) Patentinhaber: Heraeus Med GmbH, D-63450 Hanau (DE)
(72) Erfinder: Kühn, Hans-Joachim, D-65197 Wiesbaden (DE)
(74) Vertreter: Kühn, Hans-Christian

(56) Entgegenhaltungen:
- EP-A- 0 367 285
- EP-A- 0 474 069
- FR-A- 2 271 845
- US-A- 4 071 025
- US-A- 5 025 829

## Beschreibung

Die Erfindung betrifft einen Atemgasbehälter mit einem flexiblen, eine Öffnung aufweisenden Beutel mit einem im Bereich der Öffnung angeordneten Ventilgehäuse, welches ein Oberteil und ein Unterteil sowie jeweils mindestens ein Gaseinlaß- und Gasauslaßventil und einen Anschlußstutzen aufweist.

Ein derartiger Atemgasbehälter ist aus der "Wartungstafel für Ambu Beatmungsbeutel" bekannt. Hier wird der Atemgasbehälter als Sauerstoffreservoir beschrieben. Dieser Atemgasbehälter weist einen flexiblen Beutel auf, an dessen Öffnung ein Ventilgehäuse unlösbar angeordnet ist, wobei ein Unterteil des Ventilgehäuses vom Innern des Beutels her in ein Oberteil des Ventilgehäuses eingepreßt ist. An dem Oberteil ist ein Anschlußstutzen angeordnet. Das Ventilgehäuse weist Ventile zum Gaseinlaß und zum Gasauslaß auf, die zwischen Oberteil und Unterteil angeordnet sind.

Bei der Anwendung des Atemgasbehälters am Patienten besteht die Möglichkeit einer Kontamination mit Krankheitserregern, so daß der Atemgasbehälter regelmäßig gründlich gereinigt werden muß. Dies ist jedoch bei der bekannten Ausführung nur unvollständig mit vertretbarem Aufwand durchführbar, da der Beutel und das Ventilgehäuse von innen nicht zugänglich sind, ebenso wie die Ventile. Eine eventuelle Funktionsstörung des Atemgasbehälters kann weder konkret festgestellt noch behoben werden, da die einzelnen Teile für sich nicht zugänglich sind. Es ist also nicht nur durch nur mangelhafte Reinigungsmöglichkeiten (der Atemgasbehälter ist beispielsweise nicht autoklavierbar), sondern auch durch die fehlende Möglichkeit der Behebung von Funktionsstörungen erforderlich, den Atemgasbehälter relativ häufig gegen einen neuen auszutauschen.

Ausgehend von dem beschriebenen Stand der Technik liegt der Erfindung die Aufgabe zugrunde, einen Atemgasbehälter der eingangs genannten Art bereitzustellen, der zuverlässig dekontaminierbar ist und bei dem Funktionsstörungen leicht erkannt und behoben werden können.

Diese Aufgabe wird für einen Atemgasbehälter gemäß dem Oberbegriff des Anspruchs 1 dadurch gelöst, daß das Unterteil im wesentlichen in dem Beutel angeordnet ist, wobei das Unterteil innerhalb der Öffnung mindestens ein Gasauslaßventil aufweist, daß das Oberteil im wesentlichen außerhalb des Beutels angeordnet ist und mindestens ein Gaseinlaßventil und eine Anschlußöffnung sowie ein im Bereich der Öffnung angeordnetes Fixierelement mit Gasauslaßöffnungen aufweist und daß das Unterteil mit dem Oberteil lösbar verbunden ist, wobei mindestens eine Anschlußöffnung über einen Hohlraum des Oberteils mit einer Gaseinlaßöffnung des Unterteils verbunden ist. Unterteil und Fixierelement des Oberteils können tellerartig ausgebildet sein, d. h., daß ihre radiale Ausdehnung größer ist als die axiale Ausdehnung, bezogen auf eine Achse senkrecht zur Öffnung. Ein derartiger Atemgasbehälter kann nahezu ohne unzugängliche Stellen ausgebildet werden, in denen sich Keime festsetzen und einer Reinigung widerstehen können. Vorzugsweise ist der Atemgasbehälter total demontierbar, er kann daher leicht (auch in Einzelteilen) gereinigt werden. Durch die Möglichkeit der Demontage können auch Funktionsstörungen behoben werden, ohne daß der komplette Atemgasbehälter ersetzt werden muß. Die einzelnen Ventile sind leicht zugänglich und können damit auch einfach kontrolliert werden.

Zweckmäßig für einen einfachen Aufbau ist es, daß das Gasauslaßventil des Unterteils aus Gasauslaßöffnungen und einer Membran gebildet ist, die um die Gaseinlaßöffnung des Unterteils herum angeordnet sind. Auf diese Weise läßt sich die Funktion des Ventils leicht überprüfen; eine neue Membran läßt sich ggf. ohne großen Aufwand auch von Laien einsetzen.

Zweckmäßig ist es, daß die Öffnung des Beutels kreisförmig ausgebildet ist, da eine derartige Ausbildung zum einen fertigungstechnisch leicht zu realisieren ist und zum anderen durch in alle Richtungen gleichmäßige Ausbildung eine hohe Funktionssicherheit des Atemgasbehälters gewährleistet. Die Verbindung zwischen Oberteil und Unterteil erfolgt vorteilhafterweise dadurch, daß die Gaseinlaßöffnung des Unterteils und das Oberteil durch Gewinde miteinander verbunden sind. Für ein leichtes Lösen der beiden Teile voneinander ist es zweckmäßig, daß die dem Oberteil abgewandte Seite des Unterteils Griffelemente aufweist, so daß das in dem Beutel angeordnete Unterteil besser gehandhabt werden kann.

Zweckmäßig ist es weiterhin, daß das Fixierelement tellerartig ausgebildet ist und einen zum Beutel hin abgewinkelten Rand aufweist, dessen Kante am Beutel anliegt und insbesondere, daß die Gasauslaßöffnungen des Oberteils an dem abgewinkelten Rand angeordnet sind. Vorteilhaft für eine exakte Fixierung des Ventilgehäuses am Beutel ist es, daß das Unterteil innerhalb der Öffnung eine Erhebung aufweist, die an dem Rand der Öffnung anliegt, wobei der Rand der Öffnung als Wulst ausgebildet ist. Insbesondere kann die Höhe des Wulstes etwa der Höhe der Erhebung entsprechen.

Vorteilhaft ist es weiterhin, daß mindestens ein Gaseinlaßventil am Anschlußstutzen angeordnet ist, um sowohl eine einfache Gasführung als auch eine hohe Funktionssicherheit zu gewährleisten.

Der Anschlußstutzen kann in einer vorteilhaften Ausführungsform der Erfindung zwei Anschlußöffnungen mit unterschiedlichem Durchmesser aufweisen, wobei über die eine Anschlußöffnung beispielsweise Sauerstoff zugeführt werden kann, während die zweite Anschlußöffnung an einem Beatmungsbeutel angeschlossen oder (ggf. über ein Patientenventil) direkt mit einer Atemmaske verbunden werden kann. Auf diese Weise ist der Atemgasbehälter für unterschiedliche Zwecke einsetzbar (beispielsweise zur Sauerstoffanreicherung der dem Patienten über den Beatmungsbeutel zugeführten Luft, zur Sauerstofftherapie oder zur Rückatemtherapie bei einem Hyperventilationssyndrom).

Zweckmäßig für die Wartung des Atemgasbehälters ist es, daß der Beutel aus gasdichtem, autoklavierbarem Material gebildet ist bzw. daß das Ventilgehäuse im wesentlichen aus einem transparenten Material gebildet ist, wobei letzteres auch zur schnellen Erkennung von Funktionsstörungen dient.

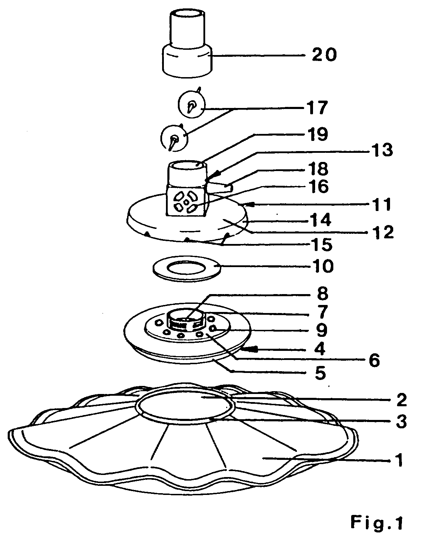

Nachfolgend wird ein Ausführungsbeispiel der Erfindung anhand einer Zeichnung näher erläutert. In der Zeichnung zeigt
Figur 1 eine Explosionsdarstellung des Atemgasbehälters und

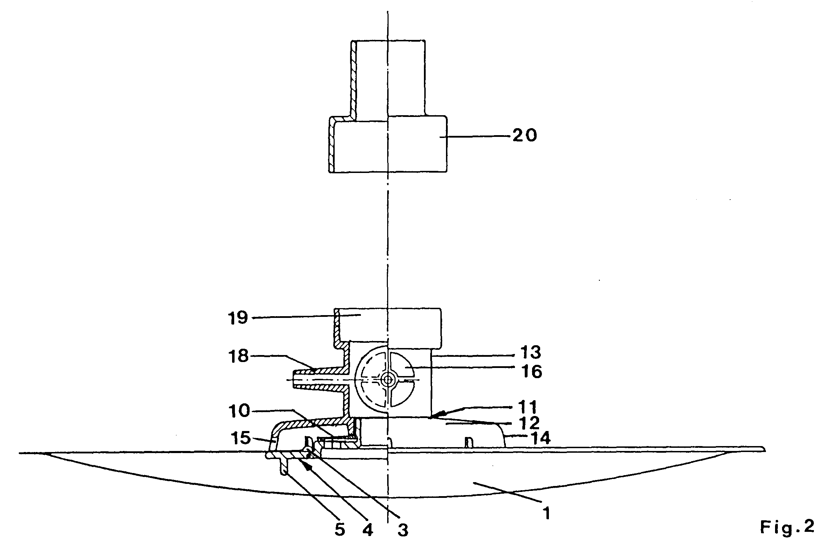

Figur 2 eine schematische Darstellung des Atemgasbehälters im Schnitt.

Der Atemgasbehälter weist einen flexiblen Beutel 1 aus silikoniertem Polyamid auf, der im wesentlichen aus zwei kreisförmigen, an ihrem Außenumfang miteinander verbundenen Teilen besteht, wobei der eine Teil in seinem Zentrum eine kreisförmige Öffnung 2 aufweist. Die Öffnung ist von einer Wulst 3 aus dem Beutelmaterial umgeben.

In dem Beutel 1 ist das Unterteil 4 des Ventilgehäuses angeordnet. Das Unterteil 4 weist an seiner Unterseite, die in das Beutelinnere hineinweist, Griffelemente 5 auf, die der besseren Handhabung des Unterteils 4 bei der Montage und Demontage dienen. Das Unterteil 4 ist im wesentlichen aus einer ebenen, kreisförmigen Scheibe aus einem transparenten Kunststoff (z. B. Polysulfon) gebildet. Im Zentrum dieser Scheibe ist eine kreisförmige Erhebung 6 angeordnet, deren Durchmesser dem Durchmesser der Öffnung 2 entspricht und deren Höhe der Höhe der Wulst 3 entspricht, so daß diese Erhebung 6 die Öffnung 2 des Beutels 1 schließt.

Im Zentrum der Erhebung 6 ist ein Gewindestutzen 7 angeordnet, der die Gaseinlaßöffnung 8 des Unterteils 4 umgibt. Auf der Erhebung sind kreisförmig um die Gaseinlaßöffnung 8 herum Gasauslaßöffnungen 9 angeordnet, auf denen eine ringförmige Membran 10 aus Silikongummi aufliegt. Dadurch bilden die auf der Erhebung 6 angeordneten Gasauslaßöffnungen 9 und die ringförmige Membran 10 das Gasauslaßventil des Unterteils 4. Die im wesentlichen ebene, um die Erhebung 6 herum angeordnete Fläche des Unterteils liegt an der Innenseite des Beutels locker an.

Auf das Unterteil 4 ist das Oberteil 11 des Ventilgehäuses aufgeschraubt. Das Oberteil 11 ist im wesentlichen aus einem Fixierelement 12 und einem Anschlußstutzen 13 gebildet. Das Fixierelement 12 weist eine tellerartige, in dem dargestellten Beispiel ebene kreisförmige Fläche auf, deren äußerer Rand 14 zum Beutel 1 hin abgewinkelt ist und an diesem locker anliegt. An der Außenkante des Randes 14 sind Gasauslaßöffnungen 15 angeordnet, durch die hindurch Gas entweichen kann, das den Beutel 1 durch das Gasauslaßventil des Unterteils 4 verläßt. Der Rand 14 liegt im Bereich des Unterteils 4 an dem Beutel 1 an, d. h., das Fixierelement 12 mit seinem Rand 14 hat einen Durchmesser, der nicht größer ist als der Durchmesser des Unterteils 4. Es ist auch denkbar, das Fixierelement 12 mit einem größeren Durchmesser auszubilden, dann jedoch ist die Funktion des Atemgasbehälters durch eventuelle mangelhafte Befestigung des Beutels 1 an dem Ventilgehäuse nicht mehr sichergestellt.

Der Anschlußstutzen 13 weist vier an dem Fixierelement angeordnete ebene Seitenflächen auf. An zwei gegenüberliegenden ebenen Seitenflächen sind Gaseinlaßventile 16 angeordnet, bei denen die Ventilöffnungen durch kreisförmige Ventilmembranen 17 aus Silikongummi im Innern des Anschlußstutzens 13 abgedeckt sind. An einer dritten ebenen Seitenfläche ist eine Anschlußöffnung 18 angeordnet, die zum Anschluß einer Sauerstoffquelle geeignet ist. Zentrisch zu dem Oberteil 11 ist an dem Anschlußstutzen 13 eine Anschlußöffnung 19 angeordnet, die zum Anschluß an einen üblichen Beatmungsbeutel geeignet ist.

Üblicherweise weisen beide Anschlußöffnungen 18; 19 unterschiedliche Durchmesser auf, um Verwechslungen während des Gebrauchs des Atemgasbehälters zu vermeiden. Dabei hat die Anschlußöffnung 19 zum Anschluß an einen Beatmungsbeutel einen wesentlich größeren Durchmesser als die Anschlußöffnung 18.

Auf die Anschlußöffnung 19 ist ein Adapter 20 aufsteckbar, über den der Atemgasbehälter direkt oder unter Zwischenschaltung eines bekannten Patientenventils an eine Beatmungsmaske angeschlossen werden kann. Ein derartiger Anschluß dient bei Zwischenschaltung eines Patientenventils der Direktversorgung des Patienten mit Sauerstoff, d. h. einer Sauerstofftherapie, während ein Anschluß des Atemgasbehälters direkt an eine Beatmungsmaske der Rückatemtherapie bei einem Hyperventilationssyndrom dient. Der Atemgasbehälter weist in dem beschriebenen Beispiel ein Maximalvolumen von etwa 1200 ml auf.

Dadurch, daß auch das Oberteil 11 des Ventilgehäuses im wesentlichen aus einem durchsichtigen Kunststoff gebildet ist, lassen sich auch grobe Verunreinigungen oder Funktionsstörungen des Atemgasbeutels schnell erkennen und durch die einfache Demontierbarkeit leicht beheben. Eventuell defekte Teile sind leicht austauschbar; die Teile des Atemgasbehälters können zuverlässig gereinigt werden, da keine unzugänglichen Stellen bestehen, an denen sich Verunreinigungen ansammeln können.

## Patentansprüche

1. Atemgasbehälter mit einem flexiblen, eine Öffnung aufweisenden Beutel, mit einem im Bereich der Öffnung angeordneten Ventilgehäuse, welches ein Oberteil und ein Unterteil sowie jeweils mindestens ein Gaseinlaß- und Gasauslaßventil und einen Anschlußstutzen aufweist, dadurch gekennzeichnet, daß das Unterteil (4) im wesentlichen in dem Beutel (1) angeordnet ist, wobei das Unterteil (4) innerhalb der Öffnung (2) mindestens ein Gasauslaßventil aufweist, daß das Oberteil (11) im wesentlichen außerhalb des Beutels (1) angeordnet ist und mindestens ein Gaseinlaßventil (16) und eine Anschlußöffnung (18; 19) sowie ein im Bereich der Öffnung (2) angeordnetes Fixierelement (12) mit Gasauslaßöffnungen (15) aufweist und daß das Unterteil (4) mit dem Oberteil (11) lösbar verbunden ist, wobei die mindestens eine Anschlußöffnung (18; 19) über einen Hohlraum des Oberteils (11) mit einer Gaseinlaßöffnung (8) des Unterteils verbunden ist.

2. Atemgasbehälter nach Anspruch 1, dadurch gekennzeichnet, daß das Gasauslaßventil des Unterteils aus Gasauslaßöffnungen (9) und einer Membran (10) gebildet ist, die um die Gaseinlaßöffnung (8) des Unterteils (4) herum angeordnet sind.

3. Atemgasbehälter nach Anspruch 1 oder 2, dadurch gekennzeichnet, daß die Öffnung (2) des Beutels (1) kreisförmig ausgebildet ist.

4. Atemgasbehälter nach einem der Ansprüche 1 bis 3, dadurch gekennzeichnet, daß die Gaseinlaßöffnung (8) des Unterteils (4) und das Oberteil (11) durch Gewinde miteinander verbunden sind.

5. Atemgasbehälter nach einem der Ansprüche 1 bis 4, dadurch gekennzeichnet, daß die dem Oberteil (11) abgewandte Seite des Unterteils (4) Griffelemente (5) aufweist.

6. Atemgasbehälter nach einem der Ansprüche 1 bis 5, dadurch gekennzeichnet, daß das Fixierelement (12) tellerartig ausgebildet ist und einen zum Beutel (1) hin abgewinkelten Rand (14) aufweist, dessen Kante am Beutel (1) anliegt.

7. Atemgasbehälter nach Anspruch 6, dadurch gekennzeichnet, daß die Gasauslaßöffnungen (15) des Oberteils (11) an dem abgewinkelten Rand (14) angeordnet sind.

8. Beutelelement nach einem der Ansprüche 1 bis 7, dadurch gekennzeichnet, daß das Unterteil (4) innerhalb der Öffnung (2) eine Erhebung (6) aufweist, die an dem Rand der Öffnung (2) anliegt, wobei der Rand der Öffnung (2) als Wulst (3) ausgebildet ist.

9. Atemgasbehälter nach Anspruch 8, dadurch gekennzeichnet, daß die Höhe der Wulst (3) etwa der Höhe der Erhebung (6) entspricht.

10. Atemgasbehälter nach einem der Ansprüche 1 bis 9, dadurch gekennzeichnet, daß mindestens ein Gaseinlaßventil (16) am Anschlußstutzen (13) angeordnet ist.

11. Atemgasbehälter nach einem der Ansprüche 1 bis 10, dadurch gekennzeichnet, daß der Anschlußstutzen (13) zwei Anschlußöffnungen (18; 19) mit unterschiedlichem Durchmesser aufweist.

12. Atemgasbehälter nach einem der Ansprüche 1 bis 11, dadurch gekennzeichnet, daß der Beutel (1) aus gasdichtem, autoklavierbarem Material gebildet ist.

13. Atemgasbehälter nach einem der Ansprüche 1 bis 12, dadurch gekennzeichnet, daß das Ventilgehäuse im wesentlichen aus einem transparenten Material gebildet ist.

## Claims

1. Breathing-gas container with a flexible bag having an opening, with a valve housing, which has an upper part and a lower part, each with at least one gas inlet valve and gas outlet valve, and a connection piece, arranged in the region of the said opening, characterized in that the lower part (4) is arranged substantially inside the bag (1) and has at least one gas outlet valve within the opening (2), in that the upper part (11) is arranged substantially outside the bag (1) and has at least one gas inlet valve (16) and connection orifice (18; 19) and also, arranged in the region of the opening (2), a fixing member (12) with gas outlet openings (15) and in that the lower part (4) is releasably connected to the upper part (11), with the at least one connection orifice (18; 19) connected to a gas outlet opening (8) via a chamber in the upper part (11).

2. Breathing-gas container according to Claim 1, characterized in that the gas outlet valve of the lower part is formed from gas outlet openings (9) and a diaphragm (10) which are disposed around the gas inlet opening (8) of the lower part (4).

3. Breathing-gas container according to Claim 1 or 2, characterized in that the opening (2) of the bag (1) is circular.

4. Breathing-gas container according to any one of Claims 1 to 3, characterized in that the gas inlet opening (8) of the lower part (4) and the upper part (11) are connected to one another by a screw joint.

5. Breathing-gas container according to any one of Claims 1 to 4, characterized in that the side of the lower part (4) facing away from the upper part (11) has grip members (5).

6. Breathing-gas container according to any one of Claims 1 to 5, characterized in that the fixing member (12) is dish-shaped and has a rim (14) angled downwards towards the bag (1) and resting on the bag (1) at its tip.

7. Breathing-gas container according to Claim 6, characterized in that the gas outlet openings (15) of the upper part (11) are arranged in the angled rim (14).

8. Bag element according to any one of Claims 1 to 7, characterized in that, inside the opening (2), the lower part (4) has an elevation (6) which rests against the edge of the opening (2), which edge is formed as a bead (3).

9. Breathing-gas container according to Claim 8, characterized in that the height of the bead (3) is about equal to the height of the elevation (6).

10. Breathing-gas container according to any one of Claims 1 to 9, characterized in that at least one gas outlet valve (16) is arranged on the connection piece (13).

11. Breathing-gas container according to any one of Claims 1 to 10, characterized in that the connection piece (13) has two connection orifices (18; 19) of different diameters.

12. Breathing-gas container according to any one of Claims 1 to 11, characterized in that the bag (1) is formed from gastight, autoclavable material.

13. Breathing-gas container according to any one of Claims 1 to 12, characterized in that the valve casing is formed substantially from transparent material.

## Revendications

1. Récipient de gaz respiratoire comportant un sac flexible présentant une ouverture, comportant un boîtier de valve agencé au niveau de l'ouverture et comprenant une pièce supérieure et une pièce inférieure ainsi qu'au moins une valve d'admission de gaz et une valve de sortie de gaz respectives et un manchon de raccordement, caractérisé en ce que la pièce inférieure (4) est disposée essentiellement dans le sac (1), ladite pièce inférieure (4) comprenant à l'intérieur de l'ouverture (2) au moins une valve de sortie de gaz, en ce que la pièce supérieure (11) est disposée essentiellement à l'extérieur du sac (1) et comprend au moins une valve d'admission de gaz (16) et une ouverture de raccordement (18 ; 19) ainsi qu'un élément de fixation (12) disposé au niveau de l'ouverture (2) et présentant des ouvertures de sortie de gaz (15), et en ce que la pièce inférieure (4) est reliée de façon détachable à la pièce supérieure (11), ladite au moins une ouverture de raccordement (18 ; 19) étant en communication avec une ouverture d'admission de gaz (8) de la pièce inférieure via une cavité de la pièce supérieure (11).

2. Récipient de gaz respiratoire selon la revendication 1, caractérisé en ce que la valve de sortie de gaz de la pièce inférieure est formée par des ouvertures de sortie de gaz (9) et par une membrane (10), lesquelles sont disposées autour de l'ouverture d'admission de gaz (8) de la pièce inférieure (4).

3. Récipient de gaz respiratoire selon l'une ou l'autre des revendications 1 et 2, caractérisé en ce que l'ouverture (2) du sac (1) est réalisée sous forme circulaire.

4. Récipient de gaz respiratoire selon l'une quelconque des revendications 1 à 3, caractérisé en ce que l'ouverture d'admission de gaz (8) de la pièce inférieure (4) et la pièce supérieure (11) sont reliées l'une à l'autre par des pas de vis.

5. Récipient de gaz respiratoire selon l'une quelconque des revendications 1 à 4, caractérisé en ce que le côté de la pièce inférieure (4), détourné de la pièce supérieure (11), comprend des éléments de préhension (5).

6. Récipient de gaz respiratoire selon l'une quelconque des revendications 1 à 5, caractérisé en ce que l'élément de fixation (12) est réalisé sous forme de plateau et comprend un rebord (14) coudé vers le sac, dont l'arête s'appuie contre le sac (1).

7. Récipient de gaz respiratoire selon la revendication 6, caractérisé en ce que les ouvertures de sortie de gaz (15) de la pièce supérieure (11) sont disposées sur le rebord coudé (14).

8. Récipient de gaz respiratoire selon l'une quelconque des revendications 1 à 7, caractérisé en ce que la pièce inférieure (4) présente à l'intérieur de l'ouverture (2) une surélévation (6) qui s'appuie contre le bord de l'ouverture (2), le bord de l'ouverture (2) étant réalisé sous forme de bourrelet (3).

9. Récipient de gaz respiratoire selon la revendication 8, caractérisé en ce que la hauteur du bourrelet (3) correspond approximativement à la hauteur de la surélévation (6).

10. Récipient de gaz respiratoire selon l'une quelconque des revendications 1 à 9, caractérisé en ce qu'au moins une valve d'admission de gaz (16) est agencée sur le manchon de raccordement (13).

11. Récipient de gaz respiratoire selon l'une quelconque des revendications 1 à 10, caractérisé en ce que le manchon de raccordement (13) présente deux ouvertures de raccordement (18 ; 19) de diamètres différents.

12. Récipient de gaz respiratoire selon l'une quelconque des revendications 1 à 11, caractérisé en ce que le sac (1) est réalisé en un matériau étanche aux gaz et susceptible d'être traité à l'autoclave.

13. Récipient de gaz respiratoire selon l'une quelconque des revendications 1 à 12, caractérisé en ce que le boîtier de valve est réalisé essentiellement en un matériau transparent.
